# EUROPEAN PATENT SPECIFICATION

(11) **EP 3 413 932 B1**
(45) Date of publication and mention of the grant of the patent: **25.03.2020**
(21) Application number: 16709820.1
(22) Date of filing: 12.02.2016
(51) Int. Cl.: A61L 15/26, A61L 15/42, A61L 15/44

(54) **A METHOD OF MANUFACTURING A PHARMACEUTICAL ACTIVE PREPOLYMER FORMULATION, FORMULATIONS OBTAINED BY THE METHOD AND USES OF THE FORMULATION**
VERFAHREN ZUR HERSTELLUNG EINER PHARMAZEUTISCH AKTIVEN PRÄPOLYMERFORMULIERUNG, DURCH DAS VERFAHREN ERHALTENE FORMULIERUNGEN UND VERWENDUNGEN DER FORMULIERUNG
PROCÉDÉ DE FABRICATION D'UNE FORMULATION DE PRÉPOLYMÈRE ACTIF PHARMACEUTIQUE, FORMULATIONS OBTENUES PAR CE PROCÉDÉ ET UTILISATIONS DE LA FORMULATION

(43) Date of publication of application: 19.12.2018
(73) Proprietor: Pharmaplast SAE, Alexandria 23512 (EG)
(72) Inventor: ATTEIA, Mamdouh, Alexandria 23512 (EG); AZIZ, Dina, Alexandria 23512 (EG); ZAKI, Mena, Alexandria 23512 (EG); ADEL, Mina, Alexandria 23512 (EG); GAD, Wassim, Alexandria 23512 (EG)
(74) Representative: Holme Patent A/S
(86) International application number: PCT/IB2016/050752
(87) International publication number: WO 2017/137808

(56) References cited:
- WO-A1-2009/033088
- WO-A2-2013/036525
- US-A1- 2008 085 949
- JOSHUA S. BOATENG ET AL: "Wound Healing Dressings and Drug Delivery Systems: A Review", JOURNAL OF PHARMACEUTICAL SCIENCES, vol. 97, no. 8, 1 August 2008 (2008-08-01) , pages 2892-2923, XP055310301, WASHINGTON, US ISSN: 0022-3549, DOI: 10.1002/jps.21210
- CHERNG JONG YUH ET AL: "Polyurethane-based drug delivery systems", INTERNATIONAL JOURNAL OF PHARMACEUTICS, ELSEVIER BV, NL, vol. 450, no. 1, 28 April 2013 (2013-04-28), pages 145-162, XP028554192, ISSN: 0378-5173, DOI: 10.1016/J.IJPHARM.2013.04.063

## Description

The present invention relates to a method of manufacturing a pharmaceutical active prepolymer formulation, in particular for subsequent foaming of the pharmaceutical active prepolymer formulation, in particular for making pharmaceutical active foam for medical wound dressings.

Foam wound dressings are very popular in advanced wound care market, and many manufacturers adds antimicrobial active ingredients to the stable foam to help treat infection or to prevent infection of wounds at high risk.

The foam is conventionally produced by mixing a toluene di-isocyanate (TDI) or methylene di-isocyanate (MDI) prepolymer with water and surfactant using dynamic mixers, and then the mixture is poured on silicone release liner and covered by another layer of silicone release liner where the space between the two layers determines the foam sheet thickness. When the foam cures, the upper release liner is removed, and then the foam sheet is dried using long conventional ovens at 100-120°C.

Another foam manufacturing method is to pour the above-mentioned mixture in siliconized cubical moulds and then the foam is left to cure and to dry in hot rooms. Finally the dry foam cubes are sliced like chips into layers with the desired thickness.

Either way, the pharmaceutical active ingredient(s) are added to an aqueous phase, often water and surfactant, with continuous agitation to ensure the homogeneity and well dispersion before mixing with the prepolymer. This poses a risk of inhomogeneity or bad distribution of the active ingredient (s) in the final foam if the agitation is not very well. Even worse is that the pharmaceutical active agents, such as silver-containing antimicrobial compounds, loose a major part of their efficiency when they come in contact with water because silver ions are released. Furthermore, these known manufacturing methods are not suitable for integration of moisture sensitive active ingredients into foam wound dressings. WO2009/033088 discloses manufacturing pharmaceutical active polyurethane formulations (polyurethane foams) for delivery of bioactive agents to wound sites.

It is a main aspect of the present invention to provide an improved way of integrating a pharmaceutical active agent into a prepolymer without loss of pharmaceutical efficiency and without noticeable gelling of the prepolymer.

It is yet an aspect of the present invention to preserve pharmaceutical activity of a pharmaceutical active agent when said agent is combined into a prepolymer.

It is a yet an aspect of the present invention to provide a pharmaceutical active prepolymer formulation that can be stored for later foaming and manufacturing of pharmaceutical active foam dressings.

It is a yet an aspect of the present invention to eliminate the need to store different prepolymer blends.

It is a yet an aspect of the present invention to provide a pharmaceutical active prepolymer formulation wherein the exposure of the workers to the various chemicals are reduced.

It is a yet an aspect of the present invention to provide a antimicrobial foam wound dressing wherein the antimicrobial agent is integrated in the prepolymer of the foam rather than poured over the more or less cured foam in a final step.

The novel and unique whereby these and other aspects are achieved according to the present invention consist in that method according to claim 1.

Within the context of the present invention the term "prepolymer" generally refers to a low molecular weight polymer that has functional groups available for reaction with a crosslinking agent. Thus the prepolymer is a monomer or system of monomers that have been reacted to prepolymerize to achieve an intermediate molecular mass state. This prepolymer formulation is a precursor capable of further polymerization.

The two main compounds controlling the type of prepolymer are the type of isocyanate and the polyol type. For urethane prepolymers, methylene di-isocyanate (MDI) and toluene diisocyanate (TDI) are the most common and preferred for the present invention. Polyols are hydroxylfunctional polyether resins usually made from the reaction of propylene oxide and/or ethylene oxide with an initiator. The typical polyols include polyester, PTMEG polyether, PPG polyether, or polycaprolactone.

Thus specifically the prepolymer is a substance formed by prereacting toluene di-isocyanate and at least one aliphatic di-isocyanate compound with one or more polyols. The prepolymer has an excess of reactive isocyanate left over to complete the final polymerization reaction. These reactive isocyanate groups that remain are measured as "% NCO". For the present invention the %NCO is from 5 to 15%.

The chemical properties of isocyanates and polyols for the formation of a prepolymer are considered known by the persons skilled in the art. Di-isocyanates compounds and polyol compounds particular suitable for use for manufacturing the pharmaceutical active prepolymer formulation according to the present invention are liquids.

Suitable di-isocyanates compounds, such as toluene di-isocyanate or methylene diisocyanate, and suitable polyol compounds, such as polyethylene glycol 1000, are commercially available from e.g. BASF Corporation, 1609 Biddle Avenue, Wyandotte, Michigan 48192.

Within the context of the present invention the term "pharmaceutical active prepolymer formulation" means a prepolymer including therein a "pharmaceutical active agent", either by admixture, such as dispersion, suspension or solution, prior to being foamed. The pharmaceutical active agent can be a solid, or be a liquid provided the water or moisture content in the liquid is less than 1.0 wt/wt%.

Within the context of the present invention the terms "integrate" and "integrated in" means that the pharmaceutical active agent becomes physically spread, such as dispersed, into the prepolymer during the formation of said prepolymer. Depending on the nature of the prepolymer compounds and pharmaceutical active agent any chemical connection between the prepolymer compounds and pharmaceutical active agent is also covered by these terms provided the functionality of the prepolymer to polymerize further and subsequently foam is not substantially negatively affected, preferably not affected at all. Preferably the integration is homogeneous.

The present application and invention specifically disclaims embodiments wherein the pharmaceutical agent is added to a cured or not-cured foam structure, e.g. by pouring or spraying, after the foam has been created. Thus addition of the pharmaceutical active agent to a foamed prepolymer is excluded from the present invention.

It is known within the art of polymer chemistry that to prevent side reactions that can lead to unintended and/or early prepolymer gelling, the reaction temperature should not increase beyond 100°C. At temperatures of 100°C to 125°C, thermally-induced formation of allophanates occurs when polyol is added to the di-isocyanate. This causes branching of prepolymers, which may lead to undesired gelling. The prepolymer is very moisture sensitive and the gelling problem is normally impossible to control if a liquid is added to prepolymer compounds. Therefore the prior art uses the above alternative ways of making pharmaceutical active wound dressings.

The water and moisture content of the pharmaceutical active agent when combined into the other prepolymer compounds is likewise established by the applicant of the present invention as being highly critical.

The combining in both of step a) and the prepolymerizing in step b) is conducted at a water or moisture content of maximum 0.8 wt/wt%, preferably maximum 0.5 wt/wt%, preferably maximum 0.3 wt/wt%, and more preferred maximum 0.1 wt/wt%.

Thereby the gelling problem is, according to the present invention, eliminated by adding a substantially dry pharmaceutical active agent to one or both of the prepolymer compounds. The dry pharmaceutical active agent can e.g. be made sufficiently dry by being subjected to a drying step prior to the combining in step a) so that the pharmaceutical active agent achieves a moisture content below the maximum water or moisture content.

The lower the water or moisture content of the pharmaceutical active agent the better, and the lower the risk of gelling.

Final polymerization that moves the pharmaceutical active prepolymer formulation towards the final curing reaction can eventually take place by adding water, optionally also a final amount of polyol and/or curative extender to the pharmaceutical active prepolymer formulation.

The water or moisture content of the pharmaceutical active agent in itself is maximum 1.0 wt/wt%, preferably maximum 0.8 wt/wt%, preferably maximum 0.5 wt/wt%, preferably maximum 0.3 wt/wt%, and more preferred maximum 0.1 wt/wt% after having been subjected to the drying step. At these moisture contents the pharmaceutical active agent is, within the scope of the present invention, understood as being "dry" and not to induce any noticeable gelling, if any at all. The maximum limit of 0.1 wt/wt% of the pharmaceutical active agent integrated in the prepolymer blend being the most "dry".

Above the claimed limits, the moisture exposure at any stage of the combining of step a) and prepolymerizing of step b) to the polyols and di-isocyanate compounds of the formulation with or without integrated pharmaceutical active agent causes gelling straightaway.

Drying can advantageously be conducted in a conventional oven or incinerator using a temperature above 300°C, e.g. for 3 hours.

Preferred pharmaceutical active agents are antimicrobial agents, e.g. antimicrobial agents useful in making wound dressings. Examples of suitable pharmaceutical active agents include, but are not limited to, one or more antimicrobial agents selected from the group comprising silver sulfadiazine, silver sodium zirconium phosphate, silver zeolite, glass silver, metallic silver, polyhexamethylene biguanide hydrochloride (PHMB), domiphen bromide, cetylpyridinium chloride, chlorohexidine, methylene blue or Gentian Violet. The most preferable pharmaceutical active agents are silver sodium zirconium phosphate or silver zeolite.

All types of antimicrobial agents can be dried in aconventional oven or incinerator, e.g. using, as stated above, the temperature above 300°C for 3 hours.

Karl Fischer Titration method was used to determine the percentage of moisture in the antimicrobial agents after drying to confirm that the drying step managed to dry the antimicrobial agents to a moisture level lower than 0.1%. For the silver-containing compounds the total silver content was measured after drying to ensure that there was no loss after drying. Volhard titration method was used and the results showed that the silver content remained within the acceptable range after drying.

The prepolymer formulation according to the present invention wherein the dry pharmaceutical active agent is integrated will be less harmful to the environment compared to conventional methods in which the foam is made first in a separate manufacturing step, and the pharmaceutical active agent is then subsequently applied, e.g. by pouring. The pharmaceutical active agent and the prepolymer compounds maintain the majority of their basic chemical and physical properties at all stages of the manufacturing method, as well as in a subsequent foaming step.

Such a final foaming step may involve adding water, and optionally a final amount of polyol and/or curative extender to the pharmaceutical active prepolymer formulation to complete the polymerization towards the final curing reaction.

When an aqueous solution of pharmaceutical agent is poured on e.g. a cured polyurethane foam structure to make a conventional pharmaceutical active wound dressing, the foam structure tend to collapse to some extent, which can induce a negative impact on absorbing and retention properties of the foam structure. This negative effect is avoided by the present invention by integration of the pharmaceutical active agent in a prepolymer prior to foaming said prepolymer. This way the foam structure can preserve a high pore volume for absorbing and retaining liquid such as exudate, as well providing a substantially sustained and homogeneous release of the pharmaceutical active agent to the wound for a certain period depending on the concentration of integrated pharmaceutical active agent in the prepolymer formulation used for making the foam.

In a preferred embodiment the pharmaceutical active agent can be included in the pharmaceutical active prepolymer formulation at a concentration of 0.1 wt/wt % up to 20 wt/wt% of the total weight of combined compounds and agents.

Adding one or more additional dry compounds selected from antioxidants, color, and surfactants is also possible.

For example can step a) include step a1) of blending the at least one polyol with one or more additional dry compounds selected from but not limited to antioxidants, color, and surfactants in a separate vessel before being combined with the remaining isocyanate compounds.

Step a) can further include step a2) of blending the aromatic and aliphatic di-isocyanate compounds together with the pharmaceutical active agent in a separate reactor before being combined with the remaining compounds. This separate reactor may be the same or different from the reactor for the final combining step a), and the blend made in step a1) may then just be added to the separate reactor of step a2) to obtain the combining of step a) .

Any or all of steps a1), a2), a) and/or b) can, according to the present invention, best be conducted at a temperature below 60°C, to further minimize the risk of premature gelling.

The duration of step b) is selected to obtain a certain degree of prepolymerization that results in a prepolymeric state of the formulation that does not take away the foamability property of the formulation at a later stage. A suitable prepolymerization duration is e.g. about 2 hours.

The pharmaceutical active prepolymer formulation can conveniently be taken from the reactor and stored under vacuum in step c) subsequent to step b) until use.

The inventive pharmaceutical active prepolymer formulation can be utilized directly to form pharmaceutical active foam without use of e.g. silver-containing compounds in an aqueous phase. This inventive foam can e.g. be provided as a pharmaceutically active foam which is applied to a liner and then cured.

The cured pharmaceutically active foam is highly beneficial for making antimicrobial wound dressings.

In a preferred embodiment manufacturing the pharmaceutical active prepolymer formulation of the present invention is done by performing the overall steps of blending the polyol compound(s) with an antioxidant in a blending vessel at around 50°C under nitrogen blanket or dry air. The di-isocyanates and the pharmaceutical active agent are charged to a cooled reactor. The polyol blend is then charged to the reactor and the temperature is maintained at 60°C for 2 hours. After 2 hours, a di-isocyanate prepolymer formulation containing an active pharmaceutical agent is formed and drained from the reactor to vacuum sucked barrels to be stored for use later on, e.g. in the manufacture of pharmaceutical active foam wound dressings.

The method according to the present invention provides an advantage over prior art methods by ensuring the consistency of pharmaceutical active agent in the final foam wound dressing as the pharmaceutical active agent is a part of the pre-polymer. In contrast, when a pharmaceutical active agent, such as a silver-containing compound, is integrated in an aqueous phase, which e.g. is poured on cured foam, the pouring solution needs continuous agitation to keep the pharmaceutical active agent dispersed in water. Any failure in agitation results in precipitation of a pharmaceutical active agent in water. desired pharmaceutical active foam product will end up having a part of the foam without a pharmaceutical active agent.

### Standardized Kirbv Bauer anti-microbial susceptibility test

A polyurethane foam test article including silver hydrogen zirconium phosphate was made of a corresponding pharmaceutical active prepolymer formulation manufactured in accordance with the method of the present invention wherein the silver hydrogen zirconium phosphate for forming the prepolymer formulation had a moisture content of 0.1 wt/wt% and 10wt/wt% active silver. The concentration of silver hydrogen zirconium phosphate in the pharmaceutical active prepolymer formulation was 13.3 w/w%.

### Test organisms:

- Staphylococcus aureus source no: ATCC® 6538™
- Pseudomonas aeruginosa source no: ATCC® 9027™

Sample size: 1 inch x 1 inch (2.54cm x 2.54cm)
Test conditions: Incubation at 37°±2°C for 18-24 hours

| Sample ID | S. aureus | P. aeruginosa |
|---|---|---|
| | Inhibition of growth around and underneath the sample | Inhibition of growth around and underneath the sample |
| 1 | 3.4 mm diameter | 3.2 mm diameter |
| 2 | 3.2 mm diameter | 3.3 mm diameter |
| 3 | 3.1 mm diameter | 3.0 mm diameter |

To summarize the invention primarily relates to a method of manufacturing a pharmaceutical active prepolymer formulation by combining in a reactor at least one polyol compound, an aromatic di-isocyanate compound in form of toluene di-isocyanate, and at least one aliphatic di-isocyanate compound, and a pharmaceutical active agent having a water or moisture content of maximum 1.0 wt/wt%.

Then the at least one polyol compound and the di-isocyanate compounds are allowed to polymerize into a precursor for subsequent polymerization, which precursor integrates the pharmaceutical active agent thereby forming the pharmaceutical active prepolymer formulation, wherein the water or moisture content in both of step a) and step b) is maximum 0.8 wt/ wt%.

When water is added to the pharmaceutical active prepolymer formulation the polymerization process reassumes under development of CO₂, to create stable foam, suitable for use in anti-microbial pharmaceutical active wound dressings.

Di-isocyanate compounds may expediently amount to about 28-34 wt/wt%, the polyol compounds to about 56-66 wt/wt% and the pharmaceutical active active agent to about 0-16 wt/wt% of the pharmaceutical active prepolymer formulation of the present invention.

## Claims

1. A method of manufacturing a pharmaceutical active prepolymer formulation that can be stored for later foaming and manufacturing of pharmaceutical active foam wound dressings, which method includes the steps of
a) combining in a reactor
- at least one polyol compound,
- an aromatic di-isocyanate compound in form of toluene di-isocyanate, and
- at least one aliphatic di-isocyanate compound, and
- a pharmaceutical active agent having a water or moisture content of maximum 1.0 wt/wt%,
and
b) in the same reactor prepolymerizing the at least one polyol compound and the at least one di-isocyanate compounds to integrate the pharmaceutical active agent to obtain the pharmaceutical active prepolymer formulation with %NCO from 5% to 15%,
wherein the water or moisture content in both of step a) and step b) is maximum 0.8 wt/wt%.

2. A method according to claim 1, wherein the aliphatic diisocyanate is methylene di-isocyanate.

3. A method according to any of claims 1 or 2, wherein the water or moisture content in any or both of step a) and step b) is maximum 0.5 wt/wt%, preferably maximum 0.3 wt/wt%, and more preferred maximum 0.1 wt/wt%.

4. A method according to any of the preceding claims 1 - 3, including a drying step wherein the pharmaceutical active agent is subjected to drying prior to the combining in step a) to achieve a water or moisture content below the maximum water or moisture content.

5. A method according to claim 4, wherein the drying step is conducted at a temperature above 300°C for at least 3 hours.

6. A method according to any of the preceding claims 1 - 5, wherein the pharmaceutical active agent is an antimicrobial agent.

7. A method according to any of the preceding claims 1 - 6, wherein the pharmaceutical active agent is one or more antimicrobial agents selected from the group comprising silver sulfadiazine, silver sodium zirconium phosphate, silver zeolite, glass silver, metallic silver, polyhexamethylene biguanide hydrochloride (PHMB), domiphen bromide, cetylpyridinium chloride, chlorohexidine, methylene blue or Gentian Violet.

8. A method according to any of the preceding claims 1 - 7, wherein the pharmaceutical active agent is included in the pharmaceutical active prepolymer formulation at a concentration of 0.1 wt/wt % up to 20 wt/wt% of the total weight of combined compounds and agents.

9. A method according to any of the preceding claims 1 - 8, wherein the method includes adding one or more additional dry compounds selected from antioxidants, color, and surfactants.

10. A method according to any of the preceding claims 1 - 9, wherein step a) includes step a1) of blending the at least one polyol with one or more additional dry compounds selected from antioxidants, color, and surfactants in a separate vessel before being combined with the remaining compounds.

11. A method according to any of the preceding claims 1 - 10, wherein step a) includes step a2) of blending the toluene di-isocyanate and the aliphatic di-isocyanate compound together with the pharmaceutical active agent in a separate reactor before being combined with the remaining compounds.

12. A method according to any of the preceding claims 1 - 11, wherein any or all of steps a1), a2), a) and/or b) is conducted at a temperature below 60°C.

13. A method according to any of the preceding claims 1 - 12, wherein step b) has a duration of about 2 hours.

14. A method according to any of the preceding claims 1 - 13, further comprising step c) subsequent to step b), wherein the pharmaceutical active prepolymer formulation is taken from the reactor and stored under vacuum.

15. A pharmaceutical active prepolymer formulation comprising a pharmaceutical active agent integrated in a prepolymer, preferably homogeneously integrated, and obtained by the method according to any of the preceding claims 1 - 14.

16. A pharmaceutical active prepolymer formulation according to claim 15, wherein the di-isocyanate compounds amount to about 28-34 wt/wt%, the polyol compounds to about 56-66 wt/wt%, and the pharmaceutical active agent to about 0-16 wt/wt%.

17. The pharmaceutical active prepolymer formulation as defined in any of the preceding claims as a composition for use in an antimicrobial wound dressing.

18. The pharmaceutical active prepolymer formulation as defined in any of the preceding claims as a composition in foamed condition for use in an antimicrobial wound dressing.

19. A pharmaceutical active wound dressing including a foamed pharmaceutical active prepolymer formulation obtained by the method according to any of the preceding claims 1 - 14.

## Patentansprüche

1. Verfahren zur Herstellung einer pharmazeutisch aktiven Präpolymer-Formulierung, die zum späteren Schäumen und zur Herstellung von pharmazeutisch aktiven Schaum-Wundauflagen aufbewahrt werden kann, wobei das Verfahren die folgenden Schritte umfasst:
a) Vereinigen in einem Reaktor
- mindestens einer Polyolverbindung,
- einer aromatischen Diisocyanatverbindung in Form von Toluoldiisocyanat,
- mindestens einer aliphatischen Diisocyanatverbindung,
- eines pharmazeutischen Aktivstoffs mit einem Wasser- oder Feuchtigkeitsgehalt von maximal 1,0 Gew.-%,
und
b) in demselben Reaktor, Präpolymerisieren der mindestens einen Polyolverbindung und der mindestens einen Diisocyanatverbindung, um den pharmazeutischen Aktivstoff zu integrieren, um die pharmazeutisch aktive Präpolymer-Formulierung mit % NCO von 5 % bis 15 % zu erhalten,
wobei der Wasser- oder Feuchtigkeitsgehalt sowohl in Schritt a) als auch in Schritt b) maximal 0,8 Gew.-% beträgt.

2. Verfahren nach Anspruch 1, wobei das aliphatische Di-Isocyanat Methylendiisocyanat ist.

3. Verfahren nach einem der Ansprüche 1 oder 2, wobei der Wasser- oder Feuchtigkeitsgehalt in einem oder beiden der Schritte a) und b) maximal 0,5 Gew.-%, vorzugsweise maximal 0,3 Gew.-%, und noch bevorzugter maximal 0,1 Gew.-% beträgt.

4. Verfahren nach einem der vorstehenden Ansprüche 1-3, umfassend einen Trocknungsschritt, bei dem der pharmazeutische Aktivstoff vor der Vereinigung in Schritt a) einer Trocknung unterzogen wird, um einen Wasser- oder Feuchtigkeitsgehalt unterhalb des maximalen Wasser- oder Feuchtigkeitsgehalts zu erreichen.

5. Verfahren nach Anspruch 4, wobei der Trocknungsschritt bei einer Temperatur von über 300 °C für mindestens 3 Stunden durchgeführt wird.

6. Verfahren nach einem der vorhergehenden Ansprüche 1-5, wobei der pharmazeutische Aktivstoff ein antimikrobieller Wirkstoff ist.

7. Verfahren nach einem der vorstehenden Ansprüche 1-6, wobei der pharmazeutische Aktivstoff ein oder mehrere antimikrobielle Wirkstoffe ist, ausgewählt aus der Gruppe, die Silbersulfadiazin, Silbernatriumzirkoniumphosphat, Silberzeolith, Glassilber, metallisches Silber, Polyhexamethylenbiguanidhydrochlorid (PHMB), Domiphenbromid, Cetylpyridiniumchlorid, Chlorhexidin, Methylenblau oder Enzianviolett umfasst.

8. Verfahren nach einem der vorhergehenden Ansprüche 1-7, wobei der pharmazeutische Aktivstoff in der pharmazeutisch aktiven Präpolymer-Formulierung in einer Konzentration von 0,1 Gew.-% bis zu 20 Gew.-% des Gesamtgewichts der kombinierten Verbindungen und Wirkstoffe enthalten ist.

9. Verfahren nach einem der vorhergehenden Ansprüche 1-8, wobei das Verfahren die Zugabe einer oder mehrerer zusätzlicher trockener Verbindungen, ausgewählt aus Antioxidantien, Farbstoffen und Tensiden, einschließt.

10. Verfahren nach einem der vorhergehenden Ansprüche 1-9, wobei Schritt a) den Schritt a1) des Vermischens des mindestens einen Polyols mit einer oder mehreren zusätzlichen trockenen Verbindungen, ausgewählt aus Antioxidantien, Farbstoffen und Tensiden, in einem separaten Gefäß vor dem Vereinigen mit den übrigen Verbindungen einschließt.

11. Verfahren nach einem der vorhergehenden Ansprüche 1-10, wobei Schritt a) den Schritt a2) des Vermischens des Toluoldiisocyanats und der aliphatischen Diisocyanatverbindung zusammen mit dem pharmazeutischen Aktivstoff in einem separaten Reaktor vor dem Vereinigen mit den übrigen Verbindungen einschließt.

12. Verfahren nach einem der vorhergehenden Ansprüche 1-11, wobei einer oder alle der Schritte a1), a2), a) und/oder b) bei einer Temperatur unter von 60 °C durchgeführt werden.

13. Verfahren nach einem der vorhergehenden Ansprüche 1-12, wobei Schritt b) eine Dauer von etwa 2 Stunden umfasst.

14. Verfahren nach einem der vorhergehenden Ansprüche 1-13, umfassend ferner Schritt c) im Anschluss an Schritt b), wobei die pharmazeutisch wirksame Präpolymer-Formulierung aus dem Reaktor entnommen und im Vakuum aufbewahrt wird.

15. Pharmazeutisch aktive Präpolymer-Formulierung, umfassend einen pharmazeutischen Aktivstoff, der in ein Präpolymer integriert ist, und vorzugsweise homogen integriert ist, und durch das Verfahren nach einem der vorhergehenden Ansprüche 1-14 erhalten wird.

16. Pharmazeutisch aktive Vorpolymer-Formulierung nach Anspruch 15, wobei die Diisocyanatverbindungen etwa 28-34 Gew.-%, die Polyolverbindungen etwa 56-66 Gew.-% und der pharmazeutische Wirkstoff etwa 0-16 Gew.-% ausmachen.

17. Die pharmazeutisch aktive Präpolymer-Formulierung gemäß einem der vorhergehenden Ansprüche als eine Zusammensetzung zur Verwendung in einem antimikrobiellen Wundverband.

18. Die pharmazeutisch aktive Präpolymer-Formulierung gemäß einem der vorhergehenden Ansprüche als eine Zusammensetzung in geschäumtem Zustand zur Verwendung in einem antimikrobiellen Wundverband.

19. Pharmazeutisch aktiver Wundverband, einschließlich einer geschäumten pharmazeutisch wirksamen Präpolymer-Formulierung, die durch das Verfahren nach einem der vorhergehenden Ansprüche 1-14 erhalten wird.

## Revendications

1. Procédé pour la préparation d'une formulation de prépolymère, active du point de vue pharmaceutique, qui peut être entreposée pour une transformation en mousse et une fabrication ultérieure de pansements en mousse actifs du point de vue pharmaceutique, ledit procédé englobant les étapes consistant à :
a) combiner, dans un réacteur :
- au moins un composé de polyol ;
- un composé de diisocyanate aromatique sous la forme du diisocyanate de toluène ; et
- au moins un composé de diisocyanate aliphatique ; et
- un agent actif du point de vue pharmaceutique possédant une teneur maximale en eau ou en humidité de 1,0 % en poids/poids ;
et
b) dans le même réacteur, soumettre à une prépolymérisation ledit au moins un composé de polyol et ledit au moins un composé de diisocyanate en vue d'intégrer l'agent actif du point de vue pharmaceutique afin d'obtenir la formulation de prépolymère active du point de vue pharmaceutique possédant un pourcentage de groupes NCO de 5 % à 15 % ;
dans lequel la teneur en eau ou en humidité à la fois à l'étape a) et à l'étape b) s'élève au maximum à 0,8 % en poids/poids.

2. Procédé selon la revendication 1, dans lequel le diisocyanate aliphatique est le diisocyanate de méthylène.

3. Procédé selon l'une quelconque des revendications 1 ou 2, dans lequel la teneur en eau ou en humidité dans l'une quelconque des étapes a) et b) ou dans les deux étapes a) et b) s'élève au maximum à 0,5 % en poids/poids ; de préférence, au maximum à 0,3 % en poids/poids ; et de manière plus préférée, au maximum à 0,1 % en poids/poids.

4. Procédé selon l'une quelconque des revendications 1 à 3, englobant une étape de séchage dans laquelle l'agent actif du point de vue pharmaceutique est soumis à un séchage avant la combinaison à l'étape a), afin d'obtenir une teneur en eau ou en humidité inférieure à la teneur maximale en eau ou en humidité.

5. Procédé selon la revendication 4, dans lequel l'étape de séchage est mise en œuvre à une température supérieure à 300 °C pendant un laps de temps d'au moins 3 heures.

6. Procédé selon l'une quelconque des revendications 1 à 5, dans lequel l'agent actif du point de vue pharmaceutique est un agent antimicrobien.

7. Procédé selon l'une quelconque des revendications 1 à 6, dans lequel l'agent actif du point de vue pharmaceutique représente un ou plusieurs agents antimicrobiens choisis parmi le groupe comprenant de la sulfadiazine d'argent, du phosphate d'argent et de sodium et de zirconium, de la zéolithe d'argent, du verre argenté, de l'argent métallique, du chlorhydrate de biguanide de polyhexaméthylène (PHMB), du bromure de domiphène, du chlorure de cétylpyridinium, de la chlorhexidine, du bleu de méthylène ou du violet de gentiane.

8. Procédé selon l'une quelconque des revendications 1 à 7, dans lequel l'agent actif du point de vue pharmaceutique est inclus dans la formulation de prépolymère active du point de vue pharmaceutique à une concentration allant de 0,1 % en poids/poids à 20 % en poids/poids du poids total des composés et des agents combinés.

9. Procédé selon l'une quelconque des revendications 1 à 8, dans lequel le procédé englobe le fait d'ajouter un ou plusieurs composés secs supplémentaires choisis parmi des antioxydants, des colorants et des agents tensioactifs.

10. Procédé selon l'une quelconque des revendications 1 à 9, dans lequel l'étape a) englobe l'étape a1) au cours de laquelle on soumet à un mélange intime ledit au moins un polyol et un ou plusieurs composés secs supplémentaires choisis parmi des antioxydants, des colorants et des agents tensioactifs dans un récipient séparé avant leur combinaison avec les composés restants.

11. Procédé selon l'une quelconque des revendications 1 à 10, dans lequel l'étape a) englobe l'étape a2) au cours de laquelle on soumet à un mélange intime le diisocyanate de toluène et le composé de diisocyanate aliphatique de manière conjointe avec l'agent actif du point de vue pharmaceutique dans un réacteur séparé avant leur combinaison avec les composés restants.

12. Procédé selon l'une quelconque des revendications 1 à 11, dans lequel l'une quelconque des étapes a1), a2), a) et/ou b) ou la totalité de ces dernières est/sont mise(s) en œuvre à une température inférieure à 60 °C.

13. Procédé selon l'une quelconque des revendications 1 à 12, dans lequel l'étape b) possède une durée d'environ 2 heures.

14. Procédé selon l'une quelconque des revendications 1 à 13, comprenant en outre l'étape c) qui fait suite à l'étape b), dans laquelle la formulation de prépolymère active du point de vue pharmaceutique est retirée du réacteur et est stockée sous vide.

15. Formulation de prépolymère active du point de vue pharmaceutique comprenant un agent actif du point de vue pharmaceutique intégré dans un prépolymère, de préférence intégré de manière homogène, et que l'on obtient par l'intermédiaire du procédé selon l'une quelconque des revendications précédentes là 14.

16. Formulation de prépolymère active du point de vue pharmaceutique selon la revendication 15, dans laquelle la quantité des composés de diisocyanate s'élève à environ 28-34 % en poids/poids ; celle des composés de polyol s'élève à environ 56-66 % en poids/poids ; et celle de l'agent actif du point de vue pharmaceutique s'élève à environ 0-16 % en poids/poids.

17. Formulation de prépolymère active du point de vue pharmaceutique telle que définie dans l'une quelconque des revendications précédentes, à titre de composition pour son utilisation dans un pansement antimicrobien.

18. Formulation de prépolymère active du point de vue pharmaceutique telle que définie dans l'une quelconque des revendications précédentes, à titre de composition dans un état transformé en mousse pour son utilisation dans un pansement antimicrobien.

19. Pansement actif du point de vue pharmaceutique englobant une formulation de prépolymère transformée en mousse, active du point de vue pharmaceutique, que l'on obtient par l'intermédiaire du procédé selon l'une quelconque des revendications précédentes 1 à 14.
